# EUROPEAN PATENT SPECIFICATION

(11) **EP 2 379 061 B1**
(45) Date of publication and mention of the grant of the patent: **14.11.2012**
(21) Application number: 09793524.1
(22) Date of filing: 17.12.2009
(51) Int. Cl.: A61K 9/26, A61K 31/4155, A61K 31/4353, A61K 9/20

(54) **Precompacted fast-disintegrating formulations of compounds with a low oral bioavailability**
Vorkompaktierte schnell zerfallende Formulierungen von Verbindungen mit geringer oraler Bioverfügbarkeit
Formulations de composés pré-comprimées à décomposition rapide à faible biodisponibilité orale

(30) Priority: 19.12.2008 EP 08172247; 19.12.2008 US 139193 P
(43) Date of publication of application: 26.10.2011
(73) Proprietor: Abbott Healthcare Products B.V., 1381 CP Weesp (NL)
(72) Inventor: MÖSCHWITZER, Jan P., 67061 Ludwigshafen (DE); WU, Yu-San, NL-1381 CP Weesp (NL); VAN TOMME, Sophie R., NL-1381 CP Weesp (NL); KUIL, Henny, NL-1381 CP Weesp (NL); KET, Aldo V., NL-1381 CP Weesp (NL); DOESBORGH-DEWIT, Lucia Maria, NL-1381 CP Weesp (NL)
(74) Representative: Jansen, Cornelis Marinus
(86) International application number: PCT/EP2009/067434
(87) International publication number: WO 2010/070057

(56) References cited:
- EP-A- 0 819 429
- EP-A- 1 070 741
- WO-A-2008/151811
- WO-A1-2005/120463
- WO-A1-2008/148731
- WO-A2-92/19227
- WO-A2-2008/148742

## Description

This invention relates to the field of pharmaceutical chemistry. Embodiments of the present invention relate to, and provide precompacted fast-disintegrating formulations of compounds with a low oral bioavailability.

### BACKGROUND

Many new drug molecules emerging from drug discovery programs have a poor bioavailability after oral administration. Bioavailability is mainly determined by two factors: first pass clearance, and absorption. Absorption is mainly influenced by the solubility of a compound, its dissolution rate, and/or its permeability. First pass clearance is a result of both intestinal and hepatic clearance (Thakker, D.R., "Strategic use of preclinical pharmacokinetic studies and in vitro models in optimizing ADME properties of lead compounds", in: Optimizing the "Drug-Like" Properties of Leads in Drug Discovery", eds. R. T Borchardt, E.H. Kerns, M. J. Hageman, D. R. Thakker, J. L. Stevens, AAPS press/Springer, 2006).

As a consequence of the above, in principle three different types of hindered bioavailability can be described. A drug molecule can have a low oral bioavailability caused by (1) a poor dissolution rate, (2) a poor solubility, or (3) a limited permeability rate. Depending on the cause, different strategies to improve oral drug absorption have been pursued.

Attempts to improve dissolution rates of poorly soluble drug molecules include formulation approaches such as micronization, nanonization or the formulation of drug molecules as solid dispersions or solid solutions.

The bioavailability of solubility rate limited drug substances can be improved by different solubilization techniques, for instance by using large quantities of surfactants, cyclodextrins, micelles, polymeric micelles, liposomes or dendrimers. To address the problem of poor permeability, the literature suggests many permeability improving substances, including mucoadhesive polymers, pH modifiers, permeation enhancers and efflux inhibitors. Permeability improving substances can be liquid, semisolid or solid excipients that are added to the solid dosage form as permeation enhancer, in cases wherein oral bioavailability is limited due to poor drug absorption.

Recent research activities were mainly focused on the development or the improvement of new formulation approaches to increase the bioavailability of problematic compounds. Fewer efforts have been undertaken to also develop final solid dosage forms that maintain or even improve the beneficial release profile of the drug formulated in relatively sophisticated ways. For the success of a selected formulation technique it is essential that the beneficial properties of the formulation are also maintained in the final solid dosage form. One standard approach to decrease disintegration time of solid dosage forms is the use of disintegrants. A disintegrant is an excipient that is added to a solid dosage form, *e.g.* a tablet or a capsule formulation, to aid in the break up of the compacted mass when it is put into a fluid environment. This is especially important for immediate release products where rapid release of drug substance is required. A disintegrant can be added to a powder blend for direct compression or encapsulation. It can also be used with products that are wet granulated (hftp://www.pformulate.com/disintegrs.htm). Many disintegrants have been described (Rowe, R.C., P.J. Shesky and S.C. Owen (editors), Handbook of Pharmaceutical Excipients, 5th Edition, Pharmaceutical Press, London, Chicago, 2006). The use of large quantities of surfactants (for solubilization approaches), or polymers (e.g. for solid dispersion), can impair the disintegration behavior of the resulting solid dosage forms significantly. Some solid dosage forms becomes "gummy", and never dissolve or disintegrate completely. Compacting formulations containing drug nanoparticles can also lead to a significant decrease in dissolution velocity by a pronounced agglomeration of the drug nanoparticles. This contrary effect is not wanted. Thus, an increased disintegration time caused by compaction of the total formulation can compensate, or even overcompensate, the positive effect of a bioavailability enhancing formulation approach. It is therefore of importance to provide formulation approaches for solid dosage forms that need an enhanced disintegration behavior.

US 4,072,535 discloses the use of a free-flowing binder-disintegrant powder material, consisting of a precompacted-starch powder, specially adapted for use in direct compression tabletting machines to produce non-friable tablets having excellent disintegration properties.

US 6,303,560 discloses the use of a compacted disintegrant granulate to improve the disintegration of compression-molded articles. In particular this patent discloses the use of a compacted disintegrant granulate for tablets, comprising at least 60-99% (w/w) cellulose, insoluble in water, 1-40% (w/w) of at least one polymeric binder, and 0-7% (w/w) of at least one liquid surfactant forming a gel in water. The goal of US 6,303,560 was to develop a formulation for direct compression with the following properties:
- free flowing uniform particles, for easy pre-blending with active ingredients, and easy loading of tabletting machines;
- a bulk density suitable to provide uniform compressibility to tablets of high hardness, low friability and uniform dosage, that remain constant in size after tabletting, and will withstand the required handling;
- inert material compatible with active ingredient and suitable for oral ingestion;
- effective disintegration rate; and
- readily available at low cost.

EP 1070741 disclosed the use of a precompacted mixture of a disintegrant and microcrystalline cellulose. This approach has some disadvantages:
- A relatively high excipient load in the final dosage form, resulting in either a large tablet size or a low drug load. The size could be reduced if pure disintegrant would be used.
- Use of a disintegrant-filler combination increases the complexity of a formulation. When roller-compaction is used to produce the filler-disintegrant mixture, one has to discard incompletely processed material to guarantee a reproducible process. This would not be the case if only a single component, like pure disintegrant, would be processed.
- Additional filler material results in a larger granulate volume, which eventually results in slower filling of the mould during the tabletting process.

No documents describe the use of a bioavailability enhancing formulation approach together with a precompacted disintegrant to enhance the bioavailability of a drug substance with low intrinsic bioavailability. The purpose of the present invention is to provide improved formulations for drug molecules with intrinsic low bioavailability.

### DISCLOSURE

This invention relates to precompacted fast-disintegrating formulations of compounds with a low intrinsic bioavailability. To solid pharmaceutical formulations for oral administration, comprising an active pharmaceutical ingredient, a hydroswelling polymer, and a precompacted granulate of a swellable excipient.

Further embodiments provide one or more formulations wherein said precompacted granulate is made by applying a compression force to the swellable excipient. Useful compression force may be evoked by an apparatus chosen from rollers under friction, roller or cube presses, extruders, ring matrix presses, and pelletizing presses.

The invention also relates, in some embodiments, to formulations as described above further comprising permeation enhancing excipients or a surfactant.

Other embodiments provide one or more formulations as described above wherein said active pharmaceutical ingredient is in the form of nanoparticles.

In preferred embodiments of the invention, the swellable excipient is 'polyvinyl pyrrolidone cross linked'; the hydroswelling polymer is chosen from the hydroxypropyl methylcelluloses HPMC E5 and HPMC E6, microcrystalline cellulose and polyvinyl pyrrolidone K12, and the surfactant is chosen from sodium dodecyl sulphate and vitamine E TPGS 1000'.

The invention also relates to a process to prepare formulations as described above, comprising the steps of:
**(i^{a})** preparing a clear solution of a hydroswelling polymer by heating water, and thereafter adding the hydroswelling polymer while stirring, until a homogeneous suspension is obtained, which is allowed to cool,
**(i^{b})** dissolving an active pharmaceutical ingredient and a weak acid into a surfactant, by stirring and heating,
**(i^{c})** mixing the solution resulting from step (i^{a}) with that resulting from step (i^{b}), and spray drying the mixture,
   or,
**(i^{d})** dissolving an active pharmaceutical ingredient and a hydroswelling polymer in a solvent, and removing the solvent by evaporation, to give an amorphous dispersion,
**(ii)** compressing a swellable excipient (disintegrant) to a tablet,
**(iii)** braking the large tablet into granules ,
**(iv)** mixing a sieve fraction of these granules with the spray-dried product of step (i^{c}), or the amorphous dispersion obtained in step (i^{d})
**(v)** pressing a tablet of the obtained mixture.

In preferred embodiments of the processes, the swellable excipient is 'polyvinyl pyrrolidone cross linked'; the hydroswelling polymer is chosen from the hydroxypropyl methylcelluloses HPMC E5 and HPMC E6, microcrystalline cellulose and polyvinyl pyrrolidone K12, the weak acid is citric acid, and the surfactant is chosen from sodium dodecyl sulphate and `vitamine E TPGS 1000'.

### DEFINITIONS

Within the context of this description, the term '**swellable excipient**' includes a material that is able to absorb a fluid medium under high volume expansion. The swelling rate can be gravimetrically determined via the water absorption capacity. The water absorption determined in this way is preferably from 500 to 2000%, including 600, 700, 800, 900, 1000, 1100, 1200, 1300, 1400, 1500, 1600, 1700, 1800, and 1900%, inclusive of all values and subranges therein between. A preferred swellable excipient is polyvinyl pyrrolidone cross linked, also referred to as polyvinyl polypyrrolidone or PVP-CL.

'**Hydroswelling polymers**', also referred to as '**water-soluble polymers**', include hydroxypropyl methylcellulose (HPMC), hydroxypropyl cellulose (HPC), hydroxyethyl cellulose (HEC), carboxymethyl cellulose (CMC). Preferable hydroswelling polymers are the hydroxypropyl methylcelluloses HPMC E5 and HPMC E6, microcrystalline cellulose and polyvinyl pyrrolidone K12. The cellulose ethers that can be used in the present invention are well known in the art and are available in pharmaceutical grades and with different average molecular weights leading to different viscosities of a solution of these cellulose ethers. Hydrophilic polymers may be characterized by their viscosities in a 2% w/w aqueous solution as low viscosity (less than about 1,000 mPas), medium viscosity (about 1,000 mPas to about 10,000 mPas) and high viscosity (greater than about 10,000 mPas). Hydrophilic hydroxypropyl methylcellulose polymers (HPMC's) are available in different viscosity grades from Dow Chemical Co. under the brand name Methocel^{®} and from Shin Etsu under Metolose^{®}. Examples of low viscosity polymers are Methocel E5^{®}, Methocel E-15LV^{®}, Methocel E50LV^{®}, Methocel K100LV^{®} and Methocel F50LV^{®}, whose 2% aqueous solutions at 25°C have viscosities of 5 mPas, 15 mPas, 50 mPas, 100 mPas and 50 mPas, respectively. Examples of medium viscosity HPMC's are Methocel E4M^{®} and Methocel K4M, whose 2% aqueous solutions at 25°C have viscosities of 4,000 mPas. Examples of high viscosity HPMC's are Methocel K15m^{®} and Methocel K100M^{®} whose 2% aqueous solutions at 25 °C have viscosities of 15,000 mPas and 100,000 mPas. Hydrophilic hydroxyethyl cellulose polymers (HEC's) are available in different viscosity grades from AQUALON under the brand name Natrosol^{®} and from Amerchol Corporation under Cellosize^{®}. Examples of low viscosity polymers are Natrosol L^{®} en Natrosol J^{®}, whose 2% aqueous solutions at 25 °C have viscosities of 10 mPas and 20 mPas, respectively. Examples of medium viscosity polymers are Natrosol G^{®} and Natrosol K^{®} whose 2% aqueous solutions at 25 °C have viscosities of 200 mPas and 1,500 mPas, respectively. Examples of high viscosity polymers are Natrosol M^{®} and Natrosol HH^{®} whose 2 % aqueous solutions have viscosities at 25 °C of 4,000 mPas and 90,000 mPas, respectively.

Citric acid is an example of a '**weak acid**', and sodium dodecyl sulphate (SDS) and 'Vitamine E TPGS 1000' *(d-a-Tocopheryl polyethylene glycol 1000 succinate)* are examples of '**surfactants**'.

The term '**intrinsic bioavailability**' describes the theoretically determined oral bioavailability based on the quantitative structure property relationship (QSPR) without considerations of formulation parameters like particle size or crystallinity (Kim, J. et al., "Improvement of bioavailability of water insoluble drugs: Estimation of intrinsic bioavailability", Korean J. Chem. Eng., 25(1), 171-175 (2008)). The term '**oral bioavailability**' describes the rate and extent of an active pharmaceutical ingredient that reaches the systemic circulation when absorbed after oral administration. It can be expressed as absolute oral bioavailability when it is compared with the corresponding plasma concentration after intravenous administration of the same API. In the context of the present invention an absolute oral bioavailability of less than 20% is regarded as '**low oral bioavailability**'.

A '**precompacted granulate**' according to the present invention refers to a granulate that is produced only by means of applying a compression force to a powder formulation of a swellable excipient resulting in a more dense agglomerate of the swellable excipient.

The term "**nanoparticle**" defines a particle comprising an active pharmaceutical ingredient with a mean size below 1000 nm. The term '**mean size**' in the framework of the present invention refers to an effective average diameter determined by dynamic light scattering methods (e.g., photocorrelation spectroscopy (PCS), laser diffraction (LD), low-angle laser light scattering (LALLS), medium-angle laser light scattering (MALLS), light obscuration methods (Coulter method, for example), rheology, or microscopy (light or electron) within the ranges set forth above). An "**effective average particle size of less than about X nm**" refers to a substance of which at least 90% of the particles have an average size of less than about X nm, when measured by the abovementioned techniques.

The term '**solid dispersion**' defines a system in a solid state (as opposed to a liquid or gaseous state) comprising at least two components, wherein one component is dispersed more or less evenly throughout the other component or components. A solid dispersion that is chemically and physically uniform or homogenous throughout or consists of one phase as defined in thermodynamics can be also referred as '**solid solution**' (WO97/044014). The solid matrix can be either crystalline or amorphous. The drug can be dispersed molecularly or exist in amorphous particles (clusters) as well as crystalline particles (solid dispersion). Examples of such a solid dispersion are the tebufelone formulation described in US 5,281,420 and the bioactive peptide formulation described in WO 2005/053727.

The term '**permeability enhancing excipient**' includes excipients known as permeability improving substances, for instance mucoadhesive polymers, pH modifiers, permeation enhancers, and efflux inhibitors.

The terms '**form**' and '**crystal form**' encompass all solid forms of the same compound. For example: polymorphs, solvates, and amorphous forms. '**Cocrystals**' are multicomponent crystals with a unique lattice: new chemical species produced with neutral compounds. '**Amorphous forms**' are non-crystalline materials with no long range order, and generally do not give a distinctive powder X-ray diffraction pattern.

To provide a more concise description, some of the quantitative expressions given herein are not qualified with the term "**about**". It is understood that whether the term "about" is used explicitly or not, every quantity given herein is meant to refer to the actual given value, and it is also meant to refer to the approximation to such given value that would reasonably be inferred based on the ordinary skill in the art, including approximations due to experimental or measurement conditions for such given value.

Throughout the description and the claims of this specification the word "**comprise**" and variations of the word, such as "comprising" and "comprises" is not intended to exclude other additives, components, integers or steps.

In the present invention '**disintegration time**' is defined as the time needed to transform a tablet into a swollen soft mass with no firm core.

A '**pharmaceutical composition**' comprises at least one active pharmaceutical ingredient (API), together with one or more pharmaceutically acceptable carriers thereof, and with or without one or more other therapeutic ingredients. The carrier(s) must be 'acceptable' in the sense of being compatible with the other ingredients of the formulation and not deleterious to the recipient thereof. The term "**composition**" as used herein encompasses a product comprising specified ingredients in predetermined amounts or proportions, as well as any product that results, directly or indirectly, from combining specified ingredients in specified amounts. In relation to pharmaceutical compositions, this term encompasses a product comprising one or more active ingredients, and an optional carrier comprising inert ingredients, as well as any product that results, directly or indirectly, from combination, complexation or aggregation of any two or more of the ingredients, or from dissociation of one or more of the ingredients, or from other types of reactions or interactions of one or more of the ingredients. In general, pharmaceutical compositions are prepared by uniformly and intimately bringing the active ingredient into association with a liquid carrier or a finely divided solid carrier or both, and then, if necessary, shaping the product into the desired formulation. The pharmaceutical composition includes enough of the active object compound to produce the desired effect upon the progress or condition of diseases. By "**pharmaceutically acceptable**" it is meant the carrier, diluent or excipient must be compatible with the other ingredients of the formulation and not deleterious to the recipient thereof.

The term "**therapeutically effective amount**" as used herein refers to an amount of a therapeutic agent to treat a condition treatable by administrating a composition of the invention. That amount includes the amount sufficient to exhibit a detectable therapeutic or ameliorative response in a tissue system, animal or human. The effect may include, for example, treating the conditions listed herein. The precise pharmaceutically effective amount for a subject will depend upon the subject's size and health, the nature and extent of the condition being treated, recommendations of the treating physician (researcher, veterinarian, medical doctor or other clinician), and the therapeutics, or combination of therapeutics, selected for administration. Thus, it is not useful to specify an exact pharmaceutically effective amount in advance.

In the framework of the present invention the terms '**biologically active substance**', '**pharmaceutically active substance**', '**drug**', '**active compound**', and '**active ingredient**' are used interchangeably to refer to a chemical substance or chemical compound that, when administered to a human or animal being, induces a pharmacological effect.

### EXAMPLE 1: COMPOUNDS USED IN FORMULATIONS

(4R)-3-(4-chlorophenyl)-4,5-dihydro-N-methyl-4-phenyl-N'-(1-piperidinylsulfonyl)-1H-pyrazole-1-carboximidamide ('**compound 1**') was synthesized as described in WO 2003/026648, and (3S)-3-[[[1-[4-[[3-(dimethylamino)propyl]methylamino]-4-oxo-(2S)-2-carboxybutyl]cyclopentyl]carbonyl]-amino]-2,3,4,5-tetrahydro-2-oxo-1H-1-benzazepine-1-acetic acid ('**compound 2**') was synthesized as described in WO 2005/030795.

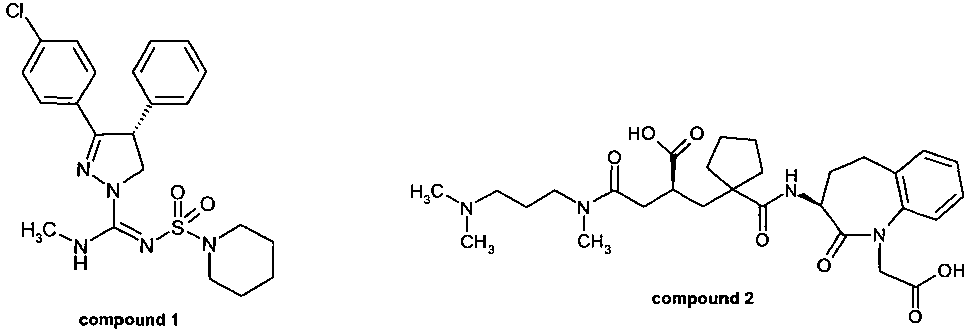

Aeropearl 300V, Aerosil^{®} 200V *(amorphous anhydrous colloidal silicon dioxide),* citric acid, hydroxypropyl methylcelluloses (*HPMC **E5** and HPMC **E6***), hydroxypropyl methylcellulose phtalate, grade 55 (*HPMC P*), Labrasol^{®}, microcrystalline cellulose, polyvinyl pyrrolidone K12, polyvinyl pyrrolidone cross linked, PRUV^{®} (*sodium stearyl fumarate*), sodium dodecyl sulphate (*SDS*) and vitamine E TPGS 1000 were obtained from commercial sources.

### EXAMPLE 2: PREPARATION OF SPECIFIC FORMULATIONS

### COMPARATIVE FORMULATION 1:

Preparation of a bioavailability enhanced oral solid dosage form (swelling excipient: polyvinyl pyrrolidone cross linked). Formulation per tablet:

| | |
|---|---|
| Poorly soluble active pharmaceutical ingredient: compound 1: | 25.0 mg |
| Vitamine E TPGS 1000: | 473.0 mg |
| Hydroxypropyl Methylcellulose (HPMC E5): | 150.0 mg |
| Citric acid: | 2.4 mg |
| Polyvinyl pyrrolidone cross linked: | 400.0 mg |
| Aeropearl 300V: | 400.0 mg |

A solution of 10% m/m HPMC E5 was prepared by first heating water to 65°C, and thereafter adding the HPMC E5 while stirring, until a homogeneous suspension was obtained. This was allowed to cool, resulting in a clear solution. Overnight, COMPOUND 1 and citric acid were dissolved into Vitamin E TPGS 1000 by stirring and heating at 60°C. This solution was mixed with the HPMC E5 solution and spray dried (INLET temperature = 160°C, OUTLET temperature = 81°C) yielding a powder wherein Vitamin E TPGS 1000 and compound 1 were thermostably embedded in a HPMC E5 matrix, further referred to as '**powder A**'. A tablet was pressed using the powder mixture, containing compound 1, Aeropearl 300V and a swelling excipient. Disintegration time: > 45 minutes.

### COMPARATIVE FORMULATION 2:

Preparation of a bioavailability enhanced oral solid dosage form (swelling excipient: polyvinyl pyrrolidone cross linked). Formulation per tablet:

| | |
|---|---|
| Poorly soluble active pharmaceutical ingredient: compound 1: | 38.0 mg |
| Vitamine E TPGS 1000: | 719.0 mg |
| Hydroxypropyl Methylcellulose (HPMC E5): | 228.0 mg |
| Citric acid: | 1.6 mg |
| Polyvinyl pyrrolidone cross linked: | 608.0 mg |

'Powder A' was prepared as described above, and was mixed with 'polyvinyl pyrrolidone cross linked'. This powder mixture was compressed into a large tablet using a hydraulic press (diameter = 52 mm, thickness ± 7 mm, compression force = 6 tons), then broken into granules, which, using a hydraulic press (length = 22.3mm, width = 10.7mm, thickness 12.0mm, compression force = 600 kg), were then pressed into tablets containing compound 1 and a swelling excipient. Disintegration time > 30 minutes.

### COMPARATIVE FORMULATION 3:

Preparation of a bioavailability enhanced oral solid dosage form (swelling excipient: polyvinyl pyrrolidone crosslinked). Formulation per tablet:

| | |
|---|---|
| Poorly soluble active pharmaceutical ingredient: compound 1: | 38.0 mg |
| Vitamine E TPGS 1000: | 719.0 mg |
| Hydroxypropyl Methylcellulose (HPMC E5): | 228.0 mg |
| Citric acid: | 1.6 mg |
| Polyvinyl pyrrolidone crosslinked: | 608.0 mg |

'Powder A', prepared as described above, was compressed into a large tablet using a hydraulic press (diameter = 52 mm, thickness ± 7 mm, compression force = 6 tons), then broken into granules. Polyvinyl pyrrolidone cross linked was mixed together with these granules and pressed into tablets using a hydraulic press (length = 22.3mm, width = 10.7mm, thickness 12.0mm, compression force = 600 kg), containing compound 1 and a swelling excipient. Disintegration time > 30 minutes.

### FORMULATION 4:

Preparation of a bioavailability enhanced oral solid dosage form (swelling excipient: polyvinyl pyrrolidone cross linked). Formulation per tablet:

| | |
|---|---|
| Poorly soluble active pharmaceutical ingredient: compound 1: | 38.0 mg |
| Vitamine E TPGS 1000: | 719.0 mg |
| Hydroxypropyl Methylcellulose (HPMC E5): | 228.0 mg |
| Citric acid: | 1.6 mg |
| Polyvinyl pyrrolidone cross linked: | 608.0 mg |

Polyvinyl pyrrolidone cross linked was compressed into a large tablet using a hydraulic press (diameter = 52 mm, thickness ± 7 mm, compression force = 6 tons), then broken into granules (precompaction via slugging). A sieve fraction (500 - 1250µm) of these granules was mixed with 'powder A', prepared as described above. A tablet was pressed using a hydraulic press (length = 22.3 mm, width = 10.7 mm, thickness 12.0 mm, compression force = 600 kg), containing compound 1 and the pre-compacted swelling excipient. The disintegration time of this improved formulation according to the present invention was approximately 5 minutes.

### FORMULATION 5:

Preparation of a fast disintegrating bioavailability enhanced oral solid dosage form (swelling excipient: polyvinyl pyrrolidone cross linked). Formulation per tablet:

| | |
|---|---|
| Poorly soluble active pharmaceutical ingredient: compound 1: | 21.0 mg |
| Vitamine E TPGS 1000: | 420.2 mg |
| Hydroxypropyl Methylcellulose (HPMC E5): | 49.3 mg |
| Citric acid: | 2.5 mg |
| Polyvinyl pyrrolidone cross linked: | 187.0 mg |

Polyvinyl pyrrolidone cross linked was compressed into a large tablet using a hydraulic press (diameter = 52 mm, thickness ± 7 mm, compression force = 6 tons), than broken into granules. A sieve fraction (500 - 1250 µm) of these granules was mixed with 'powder A', prepared as described above. A tablet was pressed using the powder mixture, containing compound 1 and the pre-compacted swelling excipient. The disintegration time of this improved formulation according to the present invention was approximately 5 minutes.

### FORMULATION 6:

Preparation of a fast disintegrating bioavailability enhanced oral solid dosage form (swelling excipient: polyvinyl pyrrolidone cross linked). Formulation per tablet:

| | |
|---|---|
| Poorly permeable active pharmaceutical ingredient: compound 2 : | 100.0 mg |
| Labrasol^{®}: | 12.5 mg |
| Hydroxypropyl Methylcellulose (HPMC E6): | 12.5 mg |
| Microcrystalline cellulose: | 102.6 mg |
| Polyvinyl pyrrolidone cross linked: | 302.6 mg |
| Aerosil 200V (amorphous anhydrous colloidal silicon dioxide): | 2.6 mg |
| PRUV^{®} (sodium stearyl fumarate): | 5.1 mg |

A solution of 10% m/m HPMC E6 was prepared by first heating water to 65°C, and thereafter adding the HPMC E6 while stirring, until a homogeneous suspension was obtained. This was allowed to cool, resulting in a clear solution. Labrasol^{®} was dispersed in the HPMC E6 solution and spray dried (INLET temperature = 145°C, OUTLET temperature = 88°C) to obtain a powder wherein Labrasol^{®} was thermostably embedded in a HPMC E6 matrix. Polyvinyl pyrrolidone cross linked was compressed into a large tablet using a hydraulic press (diameter = 52 mm, thickness ± 7 mm, compression force = 6 Tons), then broken into granules. A sieve fraction (500 - 1250 µm) of these granules was taken and mixed together with compound 2, microcrystalline cellulose, Labrasol^{®} embedded in HPMC, Aerosil^{®} and PRUV^{®}. A tablet was pressed using the powder mixture, containing compound 2 and the pre-compacted swelling excipient.

### FORMULATION 7:

Preparation of a fast disintegrating solid dispersion oral dosage form (swelling excipient: polyvinyl pyrrolidone cross linked). Formulation per tablet:

| | |
|---|---|
| Poorly soluble active pharmaceutical ingredient: compound 1: | 200.0 mg |
| Polyvinyl pyrrolidone K12: | 484.0 mg |
| Polyvinyl pyrrolidone cross linked: | 516.0 mg |

Compound 1 and polyvinyl pyrrolidone K12 were dissolved in a mixture of acetone and ethanol (3:1 v/v). The solvent was removed by flash evaporation resulting in an amorphous dispersion. Polyvinyl pyrrolidone cross linked was compressed into a large tablet using a hydraulic press (diameter = 52 mm, thickness ± 7 mm, compression force = 6 tons), then broken into granules. A sieve fraction (500 - 1250µm) of these granules was taken and mixed together with the amorphous dispersion of compound 1. A tablet was pressed using a hydraulic press (length = 22.3 mm, width = 10.7 mm, thickness 9.8 mm, compression force = 600 kg), containing compound 1 and the pre-compacted swelling excipient. The disintegration time of this improved formulation according to the present invention was approximately 2 to 3 minutes.

### FORMULATION 8:

Preparation of a fast disintegrating solid dispersion oral dosage form (swelling excipient: polyvinyl pyrrolidone cross linked). Formulation per tablet:

| | |
|---|---|
| Poorly soluble active pharmaceutical ingredient: compound 1: | 200.0 mg |
| Hydroxypropyl Methylcellulose Phtalate, grade 55 (HPMC P): | 460.0 mg |
| Polyvinyl pyrrolidone cross linked: | 516.0 mg |
| Sodium dodecyl sulphate (SDS): | 2.4 mg |

Compound 1, HPMC P and SDS were dissolved in a mixture of acetone and ethanol (3:1 v/v). The solvent was removed by flash evaporation resulting in an amorphous dispersion. Polyvinyl pyrrolidone cross linked was compressed into a large tablet using a hydraulic press (diameter = 52 mm, thickness ± 7 mm, compression force = 6 tons), then broken into granules. A sieve fraction (500 - 1250 µm) of these granules was taken and mixed together with compound 1 amorphous dispersion. A tablet was pressed using a hydraulic press (length = 22.3 mm, width = 10.7 mm, thickness 9.8 mm, compression force = 600 kg), containing compound 1 and the pre-compacted swelling excipient. The disintegration time of this improved formulation according to the present invention was approximately 30 seconds.

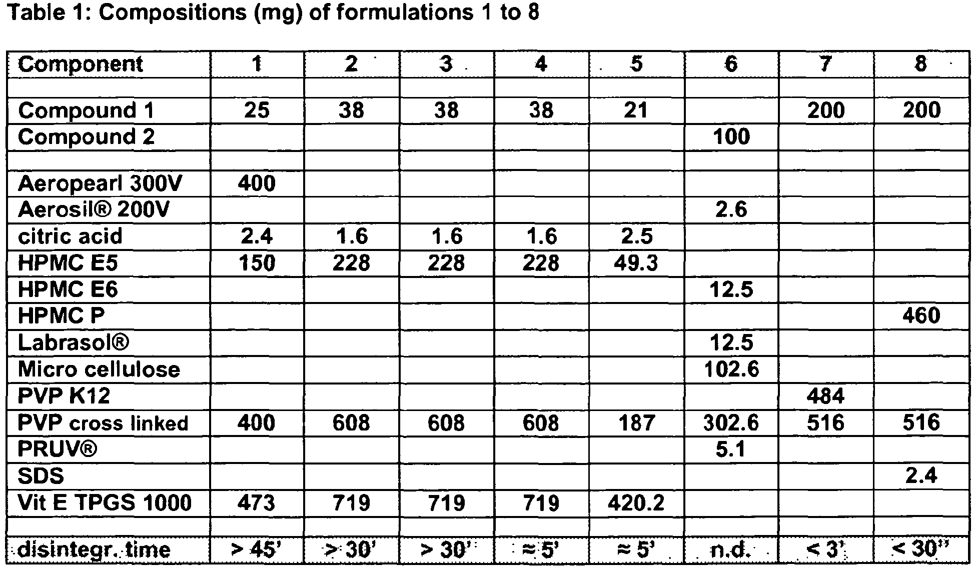

### EXAMPLE 3: MEASUREMENTS OF DISINTEGRATIONS

Disintegration tests were performed in a disintegration apparatus (PTZ-auto, Pharma-test, Hainburg, Germany) in a beaker with 800 ml purified water (temp 37± 3 °C). Disintegration is defined as that state wherein any residue of the tablet remaining on the screen of the test apparatus, or adhering to the lower surface of the disc, is a soft mass having no firm core.

### EXAMPLE 4: PRESENT FORMULATIONS COMPARED WITH THOSE OF EP 1 070 741

Different formulations have been prepared according to the procedures disclosed in EP 1 070 741 and compared to a formulation according to the present invention. The composition of the tablets is given in Table 2. The tablets 11AT, 11 BT and 11 CT have been produced according to the procedure disclosed in EP 1 070 741. Tablets 09DT2 were produced according to the present invention (Example 2, formulation 5).

In all formulations the amounts of compound 1 and PVP-cross linked were kept constant. The PVP-CUmicrocellulose(MCC) ratio, respectively 60/40, 3/97 and 10/90 in tablets 11AT, 11BT and 11CT, was varied within the two extremes disclosed in EP 1 070 741. Tablets were produced as follows: Polyvinyl pyrrolidone cross linked, with or without MCC, was compressed into a large tablet using a hydraulic press (diameter = 52 mm, thickness ± 7 mm, compression force = 6 tons) and broken into granules. A sieve fraction (500 - 1250 µm) of these granules was mixed with 'powder A', prepared as described in Example 2, and pressed into tablets using a hydraulic press.

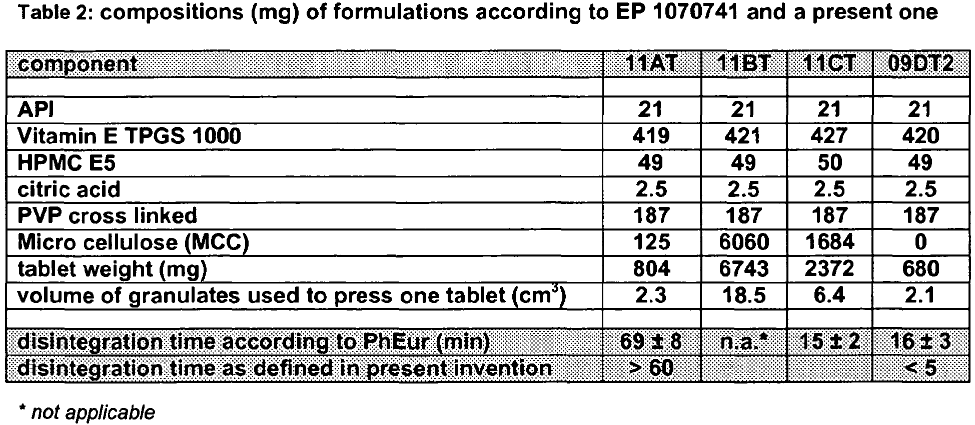

Obviously, the differences between volumes of the granulates used to press one tablet of each formulation are substantial: Use of an additional binder (MCC) results in a larger tablet size, also expected in view of the weights of the tablets (Table 2). The smallest tablet size was obtained when the tablet was produced according to the present invention.

Additionally, tablets 09AT, 09BT and 09CT were produced according to EP 1 070 741, but with a constant weight, similar to that of tablet 09DT2 (680 mg). The composition and disintegration times of these formulations are listed in Table 3.

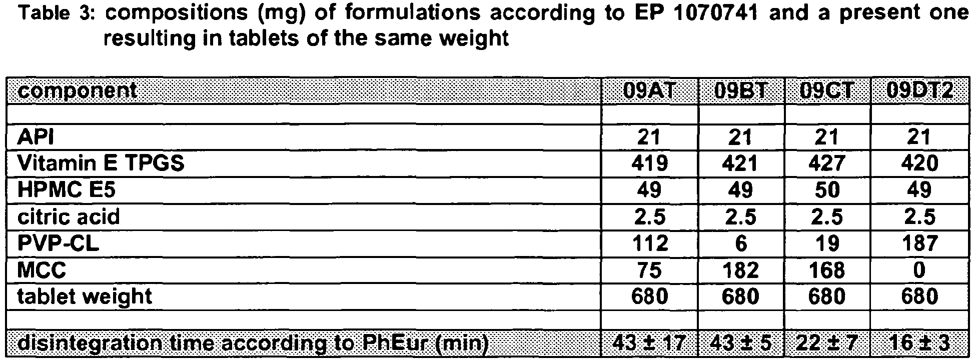

Taking into account the disintegration times of the formulations listed in tables 2 and 3, the following observations were made:
- Tablets 09DT2, produced according to the present invention, containing the same amount of disintegrant as tablets 09AT, 09BT and 09CT, had much shorter disintegration times.
- Another surprising aspect of formulations 09DT2, produced according to the present invention, was an improved reproducibility of the disintegration times.

Apart from the disintegration time, determined according to the PhEur, also disintegration behavior was determined. This was defined as 'time needed to transform a tablet into a swollen soft mass without a firm core'. The disintegration behavior of formulations 18AT and 18DT were directly compared. These tablets were chosen because of their nearly similar size. Their compositions, disintegration times and disintegration behaviors are given in table 2 (wherein tablets correspond to formulations 11AT and 09DT2, respectively).

Upon submerging the tablets into water, within 1 minute, tablets 09DT2 started to disintegrate. After 5 minutes, tablets 09DT2 were transformed into soft swollen material, contrary to tablets 11AT, which were still intact. At this point, the formulations 09DT2 were regarded as 'disintegrated' according to the definition given above. After 16 minutes, tablets 09DT2 had fully dissolved, whereas tablets 11AT were still intact.

## Claims

1. A solid pharmaceutical formulation for oral administration, comprising (1) an active pharmaceutical ingredient, (2) a hydroswelling polymer, chosen from the hydroxypropyl methylcelluloses HPMC E5 and HPMC E6, microcrystalline cellulose and polyvinyl pyrrolidone K12, and (3) a precompacted granulate of polyvinyl polypyrrolidone cross linked,

2. A pharmaceutical formulation as claimed in claim 1, wherein said precompacted granulate is made by applying a compression force to polyvinyl polypyrrolidone cross linked.

3. A pharmaceutical formulation as claimed in claim 2, wherein said compression force is evoked by an apparatus chosen from rollers under friction, roller or cube presses, extruders, ring matrix presses, and pelletizing presses.

4. A pharmaceutical formulation as claimed in any one of the claims 1-3, wherein said active pharmaceutical ingredient is in the form of nanoparticles.

5. A pharmaceutical formulation according to any one of the claims 1-4, further comprising one or more permeation enhancing excipients.

6. A pharmaceutical formulation according to any one of the claims 1-4, further comprising a surfactant.

7. A pharmaceutical formulation according to any one of the claims 1-4, further comprising one or more permeation enhancing excipients and a surfactant.

8. A pharmaceutical formulation according to claim 6 or claim 7, wherein said surfactant is chosen from sodium dodecyl sulphate and 'vitamine E TPGS 1000'

9. Process to prepare a formulation according to claim 1, comprising the steps of:
**(i^{a})** preparing a solution of a hydroswelling polymer, chosen from the hydroxypropyl methylcelluloses HPMC E5 and HPMC E6, microcrystalline cellulose and polyvinyl pyrrolidone K12, by heating water, and thereafter adding the hydroswelling polymer while stirring, until a homogeneous suspension is obtained, which is allowed to cool,
**(i^{b})** dissolving an active pharmaceutical ingredient and a weak acid into a surfactant, by stirring and heating,
**(i^{c})** mixing the solution resulting from step (i^{a}) with that resulting from step (i^{b}), and spray drying the mixture,
or,
**(i^{d})** dissolving an active pharmaceutical ingredient and the hydroswelling polymer in a solvent, and removing the solvent by evaporation, to give an amorphous dispersion,
**(ii)** compressing polyvinyl polypyrrolidone cross linked to a tablet,
**(iii)** braking the large tablet into granules ,
**(iv)** mixing a sieve fraction of these granules with the spray-dried product of step (i^{c}), or the amorphous dispersion obtained in step (i^{d})
**(v)** pressing a tablet of the obtained mixture.

## Patentansprüche

1. Pharmazeutische Feststoffformulierung zur oralen Verabreichung, umfassend (1) einen pharmazeutischen Wirkstoff, (2) ein wasserverdickendes Polymer, ausgewählt aus den Hydroxypropylmethylzellulosen HPMC E5 und HPMC E6, mikrokristalliner Zellulose und Polyvinylpyrrolidon K12 und (3) einem vorkompaktierten Granulat aus quervernetztem Polyvinylpolypyrrolidon.

2. Pharmazeutische Formulierung nach Anspruch 1, wobei das vorkompaktierte Granulat durch Anwendung einer Kompressionskraft auf quervernetztes Polyvinylpolypyrrolidon hergestellt wird.

3. Pharmazeutische Formulierung nach Anspruch 2, wobei die Kompressionskraft durch eine Vorrichtung, ausgewählt aus reibungsbeaufschlagten Rollen, Rollen- oder Würfelpressen, Strangpressen, Ringmatrixpressen und Pelletisierpressen, hevorgerufen wird.

4. Pharmazeutische Formulierung nach einem der Ansprüche 1-3, wobei der pharmazeutische Wirkstoff in der Form von Nanopartikelnvorliegt.

5. Pharmazeutische Formulierung nach einem der Ansprüche 1-4, ferner umfassend einen oder mehrere permeationsverbessernde Hilfsstoffe.

6. Pharmazeutische Formulierung nach einem der Ansprüche 1-4, ferner umfassend ein Tensid.

7. Pharmazeutische Formulierung nach einem der Ansprüche 1-4, ferner umfassend einen oder mehrere permeationsverbessernde Hilfsstoffe und ein Tensid.

8. Pharmazeutische Formulierung nach Anspruch 6 oder 7, wobei das Tensid ausgewählt ist aus Natriumdodecylsulphat und 'Vitamin E TPGS 1000'.

9. Verfahren zur Herstellung einer Formulierung nach Anspruch 1, umfassend folgende Schritte:
(i^{a}) Herstellen einer Lösung eines wasserverdickenden Polymers, ausgewählt aus den Hydroxypropylmethylzellulosen HPMC E5 und HPMC E6, mikrokristalliner Zellulose und Polyvinylpyrrolidon K12, durch Erwärmen von Wasser und anschließendem Hinzufügen des wasserverdickenden Polymers unter Rühren, bis man eine homogene Suspension erhält, die abkühlen darf,
(i^{b}) Auflösen eines pharmazeutischen Wirkstoffs und einer schwachen Säure in einem Tensid, durch Rühren und Erwärmen,
(i^{c}) Mischen der in Schritt (i^{a}) entstandenen Lösung mit der in Schritt (i^{b}) entstandenen Lösung und Sprühtrocknen des Gemischs,
oder
(i^{d}) Auflösen eines pharmazeutischen Wirkstoffs und des wasserverdickenden Polymers in einem Lösungsmittel und Entfernen des Lösungsmittels durch Verdampfung, um eine amorphe Dispersion zu erhalten, (ii) Komprimieren von quervernetztem Polyvinylpolypyrrolidon zu einer Tablette,
(iii) Brechen der großen Tablette zu Körnchen,
(iv) Mischen einer Siebfraktion dieser Körnchen mit dem sprühgetrockneten Produkt aus Schritt (i^{c}) oder der amorphen Dispersion aus Schritt (i^{d}),
(v) Pressen einer Tablette aus dem erhaltenen Gemisch.

## Revendications

1. Formulation pharmaceutique solide pour l'administration orale comprenant (1) un ingrédient pharmaceutique actif, (2) un polymère hydrogonflant, choisi parmi les hydroxypropylméthylcelluloses HPMC E5 et HPMC E6, la cellulose microcristalline et la polyvinylpyrrolidone K12, et (3) des granulés précompactés de polyvinylpolypyrrolidone réticulée.

2. Formulation pharmaceutique selon la revendication 1, où lesdits granulés précompactés sont produits par application d'une force de compression à de la polyvinylpolypyrrolidone réticulée.

3. Formulation pharmaceutique selon la revendication 2, où ladite force de compression est produite par un appareil choisi parmi les rouleaux sous frottement, les presses à rouleaux ou cubiques, les extrudeuses, les presses à matrice annulaire et les presses de pastillage.

4. Formulation pharmaceutique selon l'une quelconque des revendications 1-3, où ledit ingrédient pharmaceutique actif est sous forme de nanoparticules.

5. Formulation pharmaceutique selon l'une quelconque des revendications 1-4, comprenant en outre un ou plusieurs excipients augmentant la perméation.

6. Formulation pharmaceutique selon l'une quelconque des revendications 1-4, comprenant en outre un tensioactif.

7. Formulation pharmaceutique selon l'une quelconque des revendications 1-4, comprenant en outre un ou plusieurs excipients augmentant la perméation et un tensioactif.

8. Formulation pharmaceutique selon la revendication 6 ou la revendication 7, où ledit tensioactif est choisi parmi le dodécylsulfate de sodium et la "vitamine E TPGS 1000".

9. Procédé pour préparer une formulation selon la revendication 1, comprenant les étapes de :
(i^{a}) préparation d'une solution d'un polymère hydrogonflant, choisi parmi les hydroxypropylméthylcelluloses HPMC E5 et HPMC E6, la cellulose microcristalline et la polyvinylpyrrolidone K12, par chauffage d'eau, puis addition du polymère hydrogonflant sous agitation, jusqu'à ce qu'une suspension homogène soit obtenue, qui est mise à refroidir,
(i^{b}) dissolution d'un ingrédient pharmaceutique actif et d'un acide faible dans un tensioactif, par agitation et chauffage,
(i^{c}) mélange de la solution résultant de l'étape (i^{a}) avec celle résultant de l'étape (i^{b}), et séchage par pulvérisation du mélange,
ou
(i^{d}) dissolution d'un ingrédient pharmaceutique actif et du polymère hydrogonflant dans un solvant, et retrait du solvant par évaporation, pour obtenir une dispersion amorphe,
(ii) compression de polyvinylpolypyrrolidone réticulée en un comprimé,
(iii) fractionnement du grand comprimé en granulés,
(iv) mélange d'une fraction granulométrique de ces granulés avec le produit séché par pulvérisation de l'étape (i^{c}), ou la dispersion amorphe obtenue dans l'étape (i^{d}),
(v) compression d'un comprimé du mélange obtenu.
